# EUROPEAN PATENT APPLICATION

(11) **EP 1 411 452 A1**
(43) Date of publication of application: **21.04.2004**
(21) Application number: 01941145.3
(22) Date of filing: 21.06.2001
(51) Int. Cl.: G06F 17/60

(54) **ELECTRONIC REPORT MAKING SUPPORTING APPARATUS, METHOD, AND PROGRAM**

(71) Applicant: Kabushiki Kaisha Toshiba, Tokyo 105-8001 (JP)
(72) Inventor: NAKANO, Shinichi, Utsunomiya-shi, Tochigi 321-0967 (JP); ICHIHARA, Takashi, Otawara-shi, Tochigi 324-0047 (JP); TAKADA, Yoichi, Otawara-shi, Tochigi 324-0028 (JP)
(74) Representative: Granleese, Rhian Jane
(86) International application number: PCT/JP2001/005304
(87) International publication number: WO 2003/001421

(57) **Abstract**

A system for assisting the creation of an electronic report assists the creation of electronic report data files and holds plural report templates. An appropriate template is selected from the plural report templates according to the inspection purpose. At an arbitrary time during an inspection, plural bookmarked data files are produced. Each bookmarked data file contains information identifying inspection data of interest. After the inspection, inspection data of interest is automatically identified by the bookmarked data files. The inspection data is automatically attached to the report template.

## Description

### Technical Field

The present invention relates to a system for assisting the creation of electronic reports to assist the creation of electronic reports performed during diagnosis or after treatment (hereinafter collectively referred to as inspection), assistance of the creation of the electronic reports, and program for assisting the creation of the electronic reports.

### Background Art

Typical flow of an inspection is as follows. First, a request for an inspection is issued from the doctor in charge of a patient to the doctor and technologist in charge of the inspection. This request for the inspection includes the purpose of the inspection such as cardioangiography (CAG). At least one kind of inspection (technique) necessary to achieve the inspection purpose is determined by the doctor in charge of the patient, doctor in charge of the inspection, and technologist in charge of the inspection. For example, if the inspection purpose is an inspection for angina pectoris, four kinds of inspections (i.e., right coronary angiography, left coronary angiography, measurement of the pressures inside the ventricles of the heart, and analytical inspection of the functions of the left ventricle) are determined together with the order in which the four inspections are performed.

If the inspections are determined, devices such as catheters and drugs such as nitro used in the inspections are listed up. According to this, the devices and drugs are prepared in advance by nurses. After completion of the preparations, the inspections are actually started.

Actually, in the inspections, the plurality of inspections determined in advance are carried out according to the predetermined order. The period of each inspection is customarily called a "scene".

Attending to each inspection are the doctor in charge of the inspection, nurses supporting the doctor in charge of the inspection, a medical technologist mainly in charge of operations of the inspection apparatus such as a cardiovascular X-ray diagnostic system, and a recording person. As the inspection progresses, the recording person makes recordings of actions actually taken by the doctor in charge of the inspection and nurses. The contents of the recordings cover a wide range of items, for example, including the kind of the used catheter, the time at which the catheter was inserted into the patient, the state of the insertion, the kinds of drugs administered into the patient by nurses, their doses, the times at which the administrations were performed, the starting and ending times of the scenes, the time at which the contrast medium flowed into the region of interest, the frame number of image at this time, the time at which the contrast medium flowed out of the region of interest, and the frame number of image at this time.

Reports including inspection report, consumable report, and findings report are created after the inspections by the doctors in charge of the inspections and doctor in charge of the patient by referring to the recordings.

One of the most laborious and time-consuming tasks of the series of inspections described above is a work for creating reports, especially inspection reports. The main purpose of inspection report is to provide a list of images of interest and waveforms of interest selected from image data files and physiological phenomenon data files produced during the inspections.

For instance, images successively taken from the start to end of a scan process (referred to as the scan time) are recorded as one image data file. Usually, about 20 scans are taken in one inspection. One scan time is about 2 to 3 seconds. Therefore, about 20 image data files each having a length of about 2 to 3 seconds are produced in one inspection.

For example, if it is assumed that scans are made at a rate of 30 frames/sec, each image data file contains from 60 to 90 frames. The 20 image data files have as many as 1200 to 1800 frames in total.

The work for selecting images of interest out of such an exorbitant number of frames is not easy to perform.

In practice, the doctor in charge of the inspection memorizes a rough time when an image of interest occurred or makes a note. Furthermore, the recording person is making a record in accordance with an instruction of the doctor in charge of the inspection. Therefore, images around that time are played back and a selection is made from them. Although it is not necessary to reproduce all the image data files, it is not unavoidable that very much labor and long time be required.

These similarly take place when waveforms of interest are selected from vital sign data files of inside pressures, electrocardiograms, etc.

### Disclosure of Invention

It is an object of the present invention to improve the efficiency of the creation of an electronic report.

A system for assisting creation of an electronic report in accordance with the invention is constructed to assist the creation of an electronic report data file about at least one inspection data file of interest or a part thereof included in a plurality of inspection data files produced by inspections using a medical imaging diagnostic system or medical measuring instrument. A certain report template data file complying with the inspection purpose is selected from a plurality of report template data files containing layout information in the inspection data files. At least one bookmarked data file containing information identifying the inspection data file of interest or part thereof is produced during the inspection. After the inspection, the inspection data file of interest or part thereof is selectively taken in from the plural inspection data files based on the bookmarked data files. The inspection data file of interest or part thereof is attached to the electronic report data file as a reference file based on the layout information in the certain report template data file. The produced electronic report data file is output or stored together with the reference file.

### Brief Description of Drawings

FIG. 1 is a diagram showing a LAN configuration including a system for assisting creation of electronic reports according to an embodiment of the present invention.
FIG. 2 is a structural diagram of a system for assisting creation of electronic reports according to an embodiment of the invention.
FIG. 3 is a structural diagram of the bookmarked data file-generating portion of FIG. 2.
FIG. 4 is a structural diagram of the electronic report data file-generating portion of FIG. 2.
FIG. 5 is a diagram showing a table of the correspondences between inspection purposes and templates managed by the inspection-managing portion of FIG. 2.
FIG. 6 is a diagram showing an example of inspection report template kept in storage in the report template database of FIG. 2.
FIG. 7 is a diagram showing an example of device report template kept in storage in the report template database of FIG. 2.
FIG. 8 is a diagram showing an example of device report template for editing, the template being kept in storage in the report template database of FIG. 2.
FIG. 9 is a diagram showing an example of drug report template kept in storage in the report template database of FIG. 2.
FIG. 10 is a diagram showing one example of diagnostic procedure template kept in storage in the device set/drug set/diagnostic procedure database of FIG. 2.
FIG. 11 is a diagram illustrating output matter delivered by an electronic report creation-assisting system according to the present embodiment at stages of before inspection, during inspection, and after inspection.
FIG. 12 is a diagram showing an example of chance of producing a bookmarked data file during inspection in the present embodiment.
FIG. 13A is a diagram showing the data structure of a bookmarked data file for scene, the data file being created by the bookmarked data file-creating portion of FIG. 3.
FIG. 13B is a diagram showing the data structure of a bookmarked data file for image, the data file being created by the bookmarked data file-creating portion of FIG. 3.
FIG. 13C is a diagram showing the data structure of a bookmarked data file for device, the data file being created by the bookmarked data file-creating portion of FIG. 3.
FIG. 13D is a diagram showing the data structure of a bookmarked data file for drug, the data file being created by the bookmarked data file-creating portion of FIG. 3.
FIG. 13E is a diagram showing the data structure of a bookmarked data file for measurement, the data file being created by the bookmarked data file-creating portion of FIG. 3.
FIG. 14 is a flowchart illustrating a procedure of producing a bookmarked data file by means of the bookmarked data file-generating portion of FIG. 2.
FIG. 15 is a diagram illustrating a speech recognition tool used by the speech recognition portion of FIG. 3.
FIG. 16 is a flowchart illustrating a procedure of producing an electronic report data file by means of the electronic report data file-generating portion of FIG. 2.
FIG. 17 is a diagram illustrating an unedited inspection report in the present embodiment.
FIG. 18 is a diagram illustrating an unedited device report in the present embodiment.
FIG. 19 is a diagram illustrating an unedited drug report in the present embodiment.
FIG. 20 is a diagram illustrating an unedited execution recording report in the present embodiment.
FIG. 21 is a diagram illustrating an inspection report for editing in the present embodiment.
FIG. 22 is a diagram illustrating a device report for editing in the present embodiment.
FIG. 23 is a diagram illustrating an edited device report in the present embodiment.
FIG. 24 is a diagram illustrating an edited drug report in the present embodiment.
FIG. 25 is a diagram illustrating an edited execution recording report in the present embodiment.
FIG. 26 is a diagram illustrating a sorted execution recording report in the present embodiment.

### Best Mode for Carrying Out the Invention

The present invention is hereinafter described in detail using the preferred embodiments with reference to the drawings.

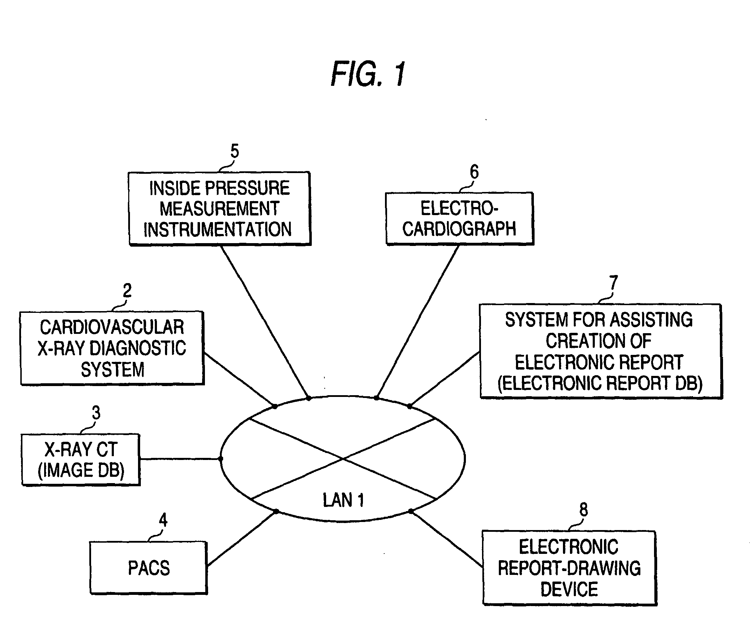

As shown in FIG. 1, a system 7 for assisting creation of an electronic report according to the present embodiment constitutes a LAN system or WAN system together with a LAN network 1 (or WAN network), a cardiovascular X-ray diagnostic system 2, an X-ray computed tomography system (X-ray CT scanner) 3, an inside pressure-measuring instrument 5, an electrocardiograph 6, a medical picture archiving and communications system (PACS) 4, an electronic report-drawing device or apparatus 8 such as a medical workstation, an HIS (hospital information system) (not shown), and a radiological department information management system (not shown).

In FIG. 1, the cardiovascular X-ray diagnostic system 2 and X-ray CT scanner 3 are shown as examples of medical imaging systems. However, medical imaging systems are not limited to them. They may also be magnetic resonance imager (MRI), ultrasonic diagnostic system, or nuclear medical diagnostic system (gamma camera, SPECT, or PET). The inside pressure-measuring instrument 5 and electrocardiograph 6 are shown as examples of physiological phenomenon-measuring instruments. The physiological phenomenon-measuring instruments are not restricted to them. They may also be electroencephalograph, phonocardiograph, electromyograph, respirometer, sphygmomanometer, blood flow meter, clinical thermometer, and other instruments for measuring various physiological phenomena (vital signs). These medical imaging diagnostic instruments and physiological phenomenon-measuring instruments are collectively defined as the inspection equipment. Inspection data is data regarding images and waveforms produced by the inspection equipment. A sequence of frames produced successively from start to end of a scan or measurement is treated as one file.

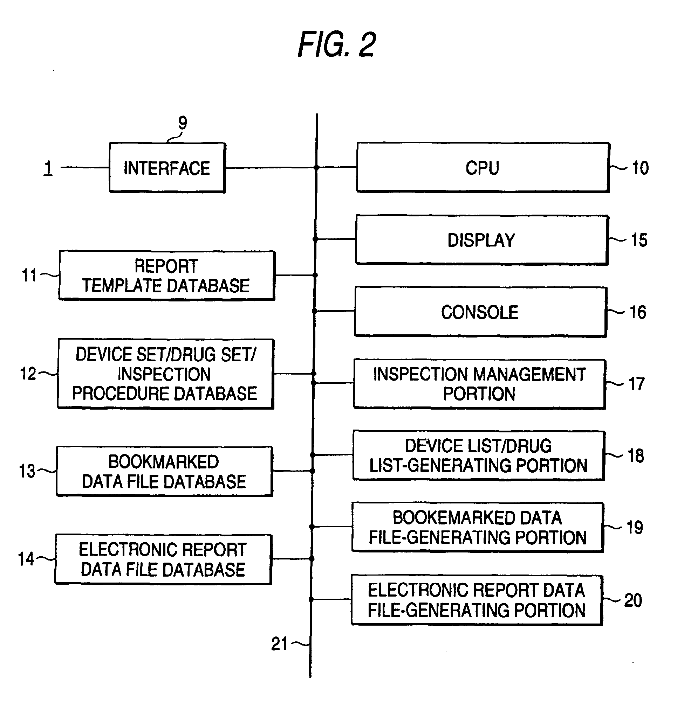

As shown in FIG. 2, the system 7 for assisting creation of an electronic report is connected with the LAN network 1 via a LAN interface 9. A database 11 for report templates, a database 12 for device set/drug set/inspection procedure, a database 13 for bookmarked data files, and a database 14 for electronic report data files are connected via a data control bus 21 with a CPU 10 acting as a controlling and arithmetic center in the electronic report creation-assisting system 7. In practice, these databases 11-14 are made of one or more storage units.

Furthermore, a display unit 15, a console 16, an inspection management portion 17, a device list/drug list-generating portion 18, a bookmarked data file-generating portion 19, and an electronic report data file-generating portion 20 are connected with the CPU 10 via the data control bus 21. In practice, inspection management, generation of device list/drug list, generation of a bookmarked data file, and generation of an electronic report data file are offered by a computer operated by program codes. The inspection management portion 17, device list/drug list-generating portion 18, bookmarked data file-generating portion 19, and electronic report data file-generating portion 20 are offered as subsets of the program codes.

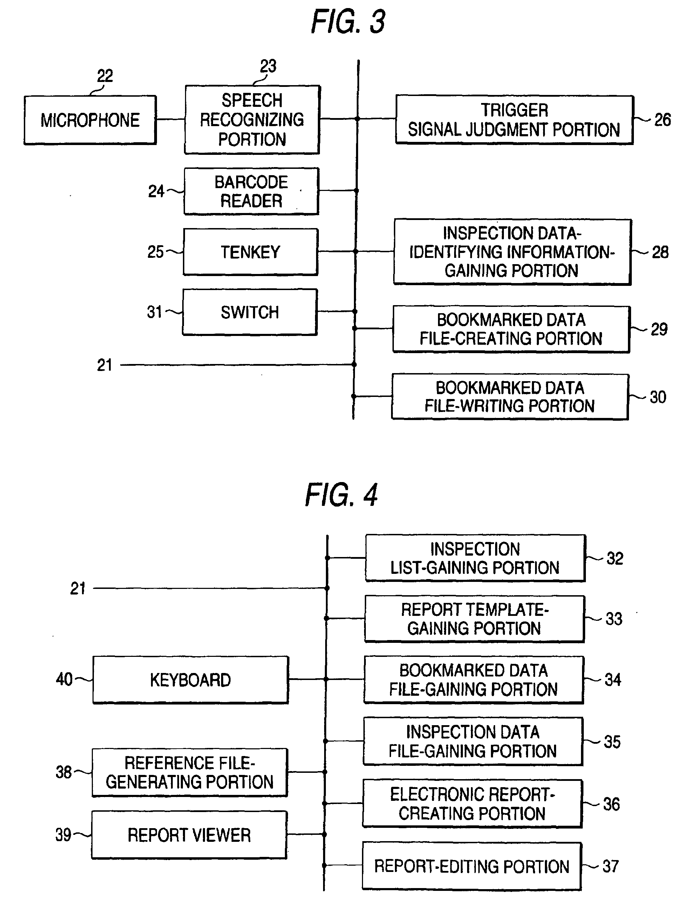

As shown in FIG. 3, the bookmarked data file-generating portion 19 is fitted with a microphone 22, a barcode reader 24, a tenkey 25, and a switch 31 as man-machine interfaces for entering information necessary to create bookmarked data files. The bookmarked data file-generating portion 19 is fitted with a trigger signal judgment portion 26, an inspection data-identifying information-gaining portion 28, a bookmarked data file-creating portion 29, and a bookmarked data file-writing portion 30.

As shown in FIG. 4, the electronic report data file-generating portion 20 is made up of an inspection list-gaining portion 32, a report template-gaining portion 33, a bookmarked data file-gaining portion 34, an inspection data file-gaining portion 35, an electronic report-creating portion 36, a report-editing portion 3, a reference file-generating portion 38, a report viewer 39, and a keyboard 40.

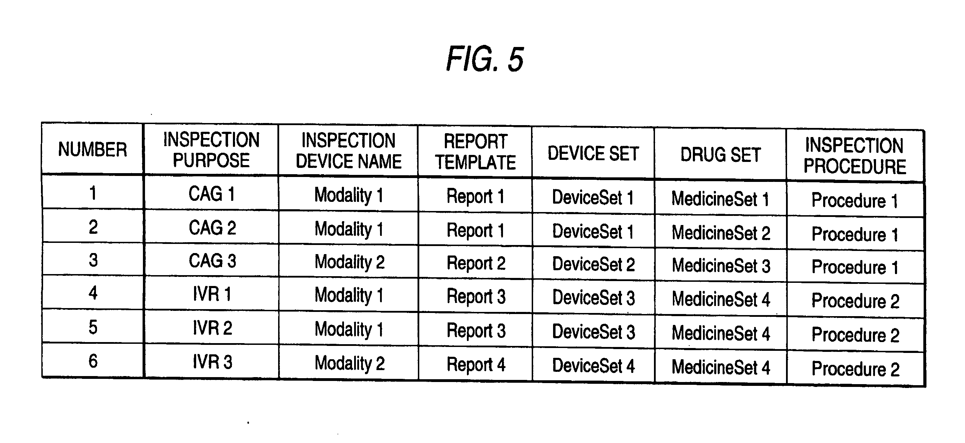

FIG. 5 shows a table of correspondences between inspection purposes and templates managed by the inspection management portion 17. In the inspection management table, identification codes of the inspection equipment, path to each report template data file, path to each device set data file, path to each drug set data file, and path to each inspection procedure data file are correlated to each of plural inspection purposes such as inspection for angina pectoris (CAG1).

This inspection management table is constructed appropriately. That is, the inspection equipment, report templates, device sets, drug sets, and diagnostic procedures are appropriately combined according to the inspection purpose. Using the inspection management table, one or more inspection devices used for inspections necessary to achieve the inspection purpose can be identified from the inspection purpose. An access is made to the database 12 according to paths identified from the inspection purpose. Thus, a device data file describing information about devices such as catheters used in the inspection that are necessary to achieve the inspection purpose, a drug data file describing information about drugs used in this inspection, and an inspection procedure data file describing the procedure of the inspection can be gained.

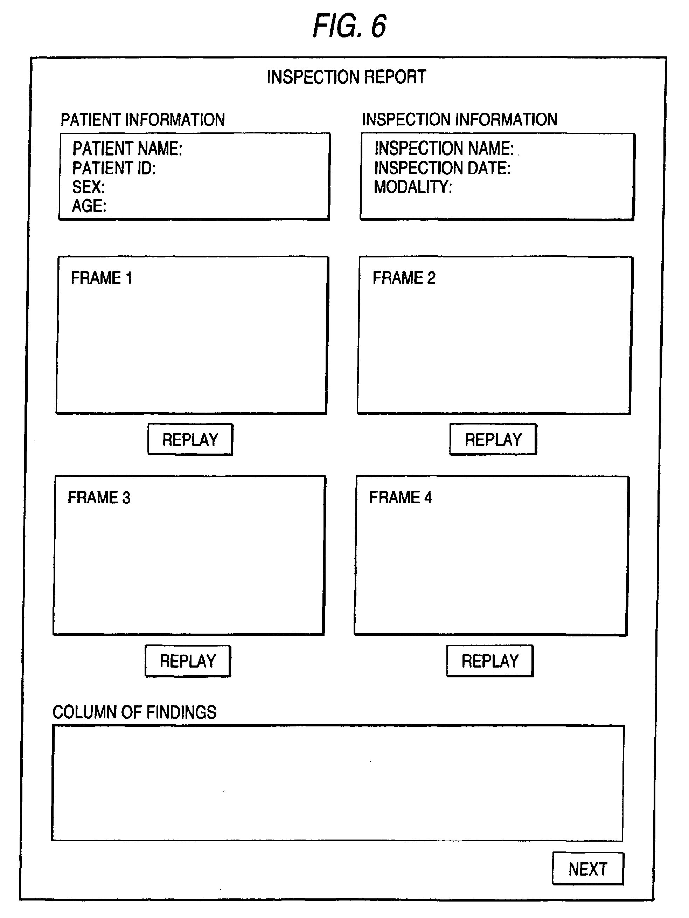

FIG. 6 shows an example of template of an inspection report. The inspection report contains patient information, inspection information, image information, and findings information. The format of this inspection report is set by the inspection report template data file. That is, layout information defining character fonts, character sizes, page format, frame lines, positions into which the character information in the aforementioned patient information, inspection information, and findings information are inserted, positions to which images are attached, their ranges, and the number is contained in the inspection report template.

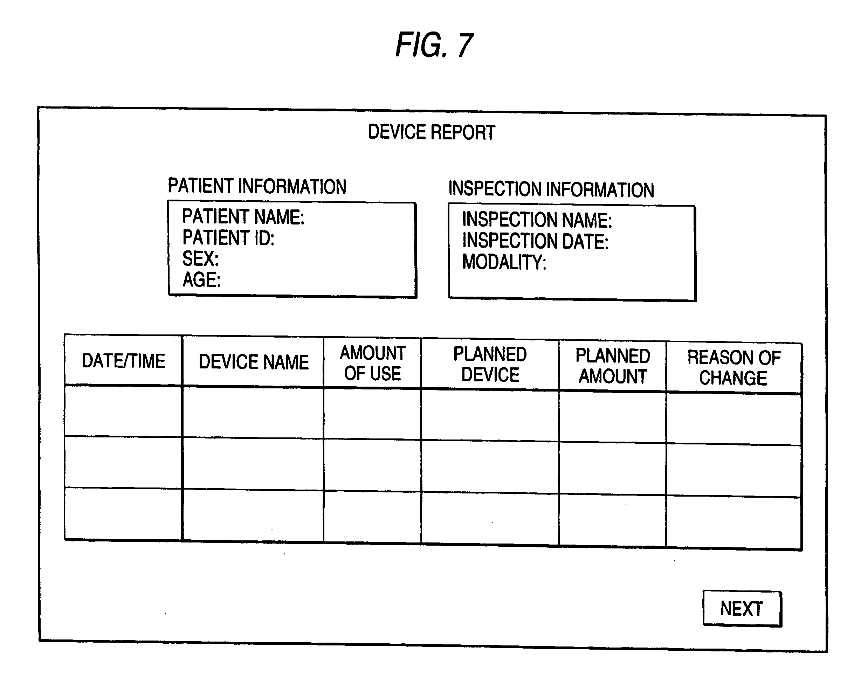

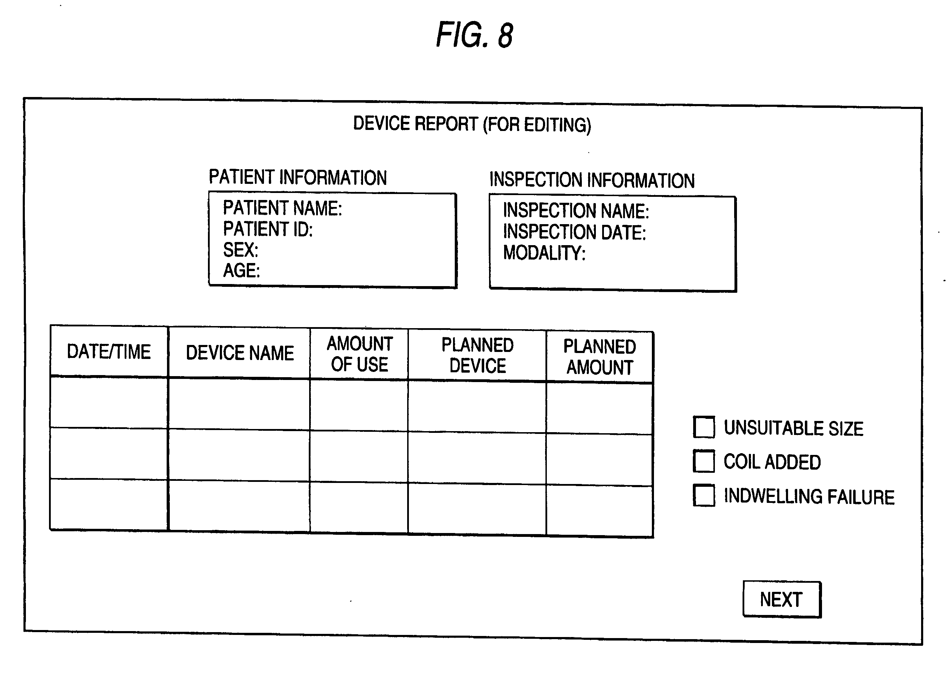

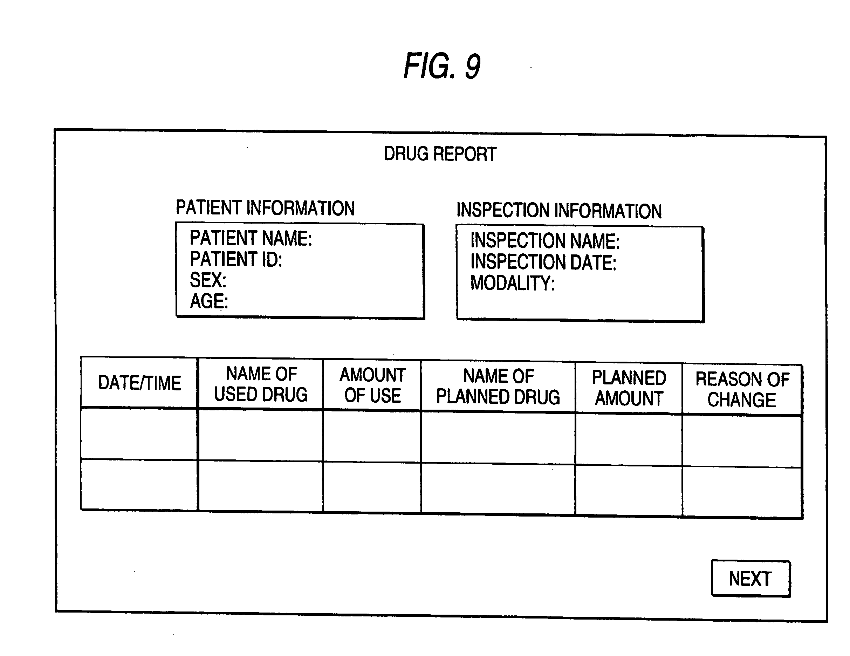

FIGS. 7, 8, and 9 show examples of device report template, device report template for editing, and drug report template, respectively.

Similarly to the inspection report templates, the report templates contain layout information defining character fonts, character sizes, page format, frame lines, positions at which the aforementioned patient information, inspection information, and information about devices and drugs are attached, and the number of them.

The user side can customize the above-described correspondence table and various report tables at will.

The operation of the present embodiment is next described together with the whole flow of the inspection. The inspection starts by being triggered by an inspection request issued by the doctor in charge of the patient. This inspection request contains the inspection purpose, as well as the patient information.

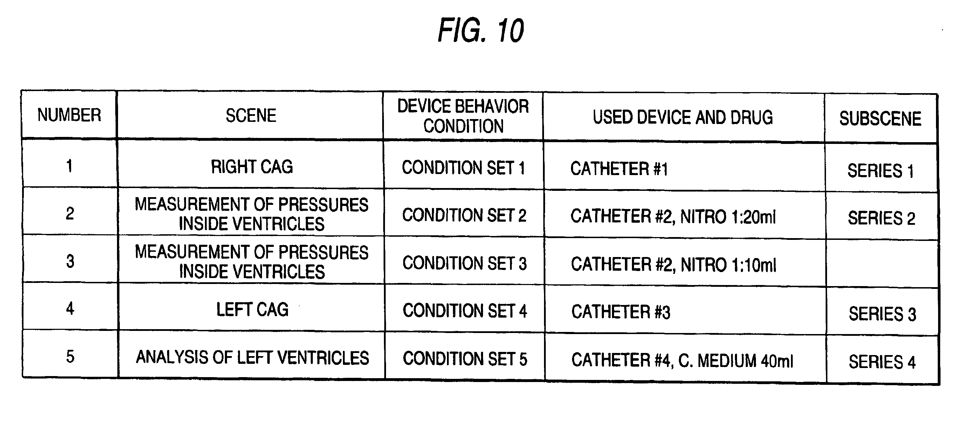

If the inspection request is entered via the console 16, together with the patient information and inspection purpose, an identification number (inspection UID) of this inspection is issued by the inspection management portion 17. The device list/drug list-generating portion 18 gains a device data file, a drug data file, and an inspection procedure data file shown in FIG. 10 from the database 12 according to device data file path data and drug data file path data correlated to the inspection purpose on the correspondence table.

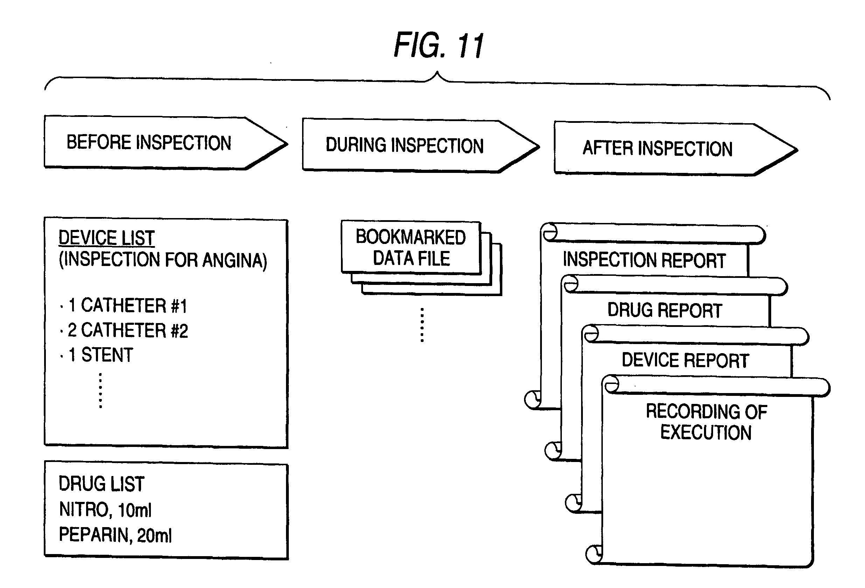

And, the device list/drug list-generating portion 18 creates a device list and a drug list illustrated in FIG. 11, based on the gained device data file and drug data file. The device list and drug list are displayed on the display unit 15 and printed out. The staff such as nurses refers to the lists. Thus, devices and drugs necessary for the inspection are efficiently prepared without omission. Furthermore, an inspection schedule table is created by the generating portion 18 based on the inspection procedure data file. The inspection schedule table permits the doctor in charge of the inspection to check the inspection schedule.

Then, the inspection is carried out in practice. During the inspection, a table of inspection steps is created by the inspection management portion 17 based on the inspection procedure data file shown in FIG. 10 and displayed on the display unit 15 or on other display unit installed in the examination room.

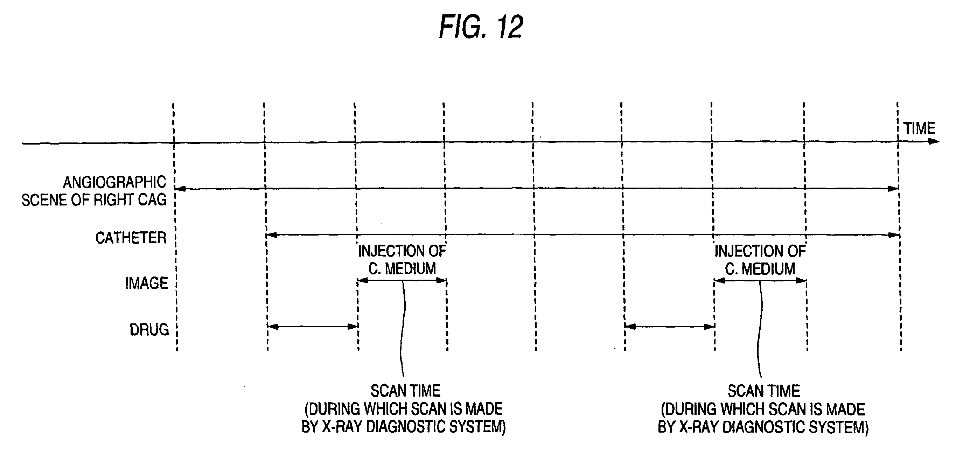

Usually, an inspection is divided into plural scenes according to difference in inspection category. Furthermore, each scene is divided into plural subscenes according to difference in working category. For example, as shown in FIG. 12, in a right CAG (coronary angiography) inspection scene, the scan (angiographic) subscene is repeated normally while inserting a catheter. In each scan subscene, images are taken in succession. Each period is referred to as a scan time. Usually, immediately after each scan subscene, contrast medium is injected. The names of these scenes and the names of the subscenes are arranged in their order, and are displayed as a step table together with devices, names of drugs, and planned amounts used in the subscenes.

The inspection management portion 17 causes the present scene and subscene to be displayed inverted as the inspection progresses. As described later, when the scene and subscene are switched, a trigger signal for urging generation of a bookmarked data file for each type of scene is produced. In synchronism with the trigger signal for each type of scene, the scene and subscene to be displayed inverted can be switched.

The operation for producing bookmarked data files is next described by referring to FIG. 12. In the present embodiment, five types for scene, device, drug, image, and measurement, respectively, are prepared as bookmarked data files. The doctor in charge of the inspection and nurses can produce these bookmarked data files at arbitrary times. Typically, the bookmarked data file for scene is produced at the start of the scene. The bookmarked data file for drugs is produced when drugs and contrast medium are administered. The bookmarked data file for devices is produced when a device such as a catheter is used. The bookmarked data file for image is produced when an image of interest is displayed. The bookmarked data file for measurement is produced when a peculiar physiological waveform appears.

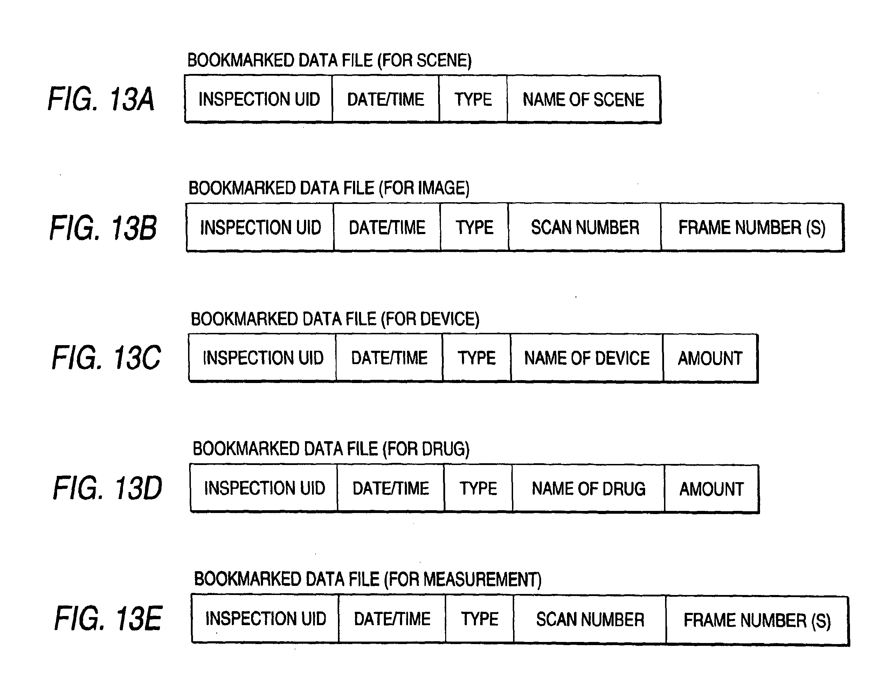

FIG. 13A shows the data structure of the bookmarked data files for scenes. FIG. 13B shows the data structure of the bookmarked data files for images. FIG. 13C shows the data structure of the bookmarked data files for devices. FIG. 13D shows the data structure of the bookmarked data files for drugs. FIG. 13E shows the data structure of the bookmarked data files for measurement. Items common to every type are inspection UID, date/time at which instructions given from the doctor in charge of the inspection or nurses to create bookmarked data files are entered, and types. Furthermore, there are individual items. That is, bookmarked data files for scenes contain scene names. Bookmarked data files for images contain scan numbers and frame number or numbers. Bookmarked data files for devices contain device names and their amounts of use. Bookmarked data files for drugs contain drug names and their amounts of use. Bookmarked data files for measurement contain scan numbers and frame number or numbers.

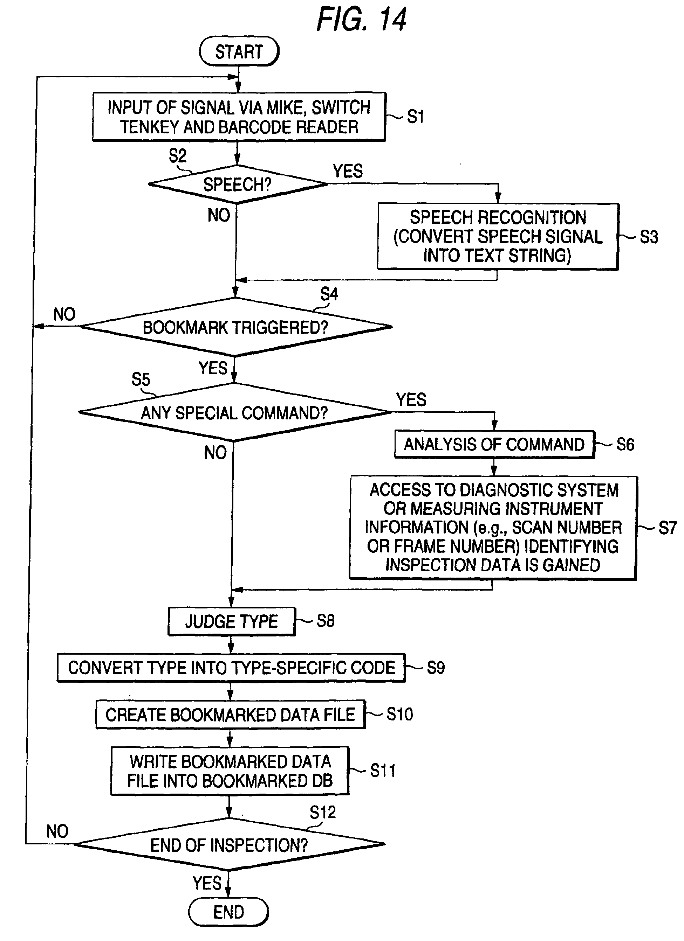

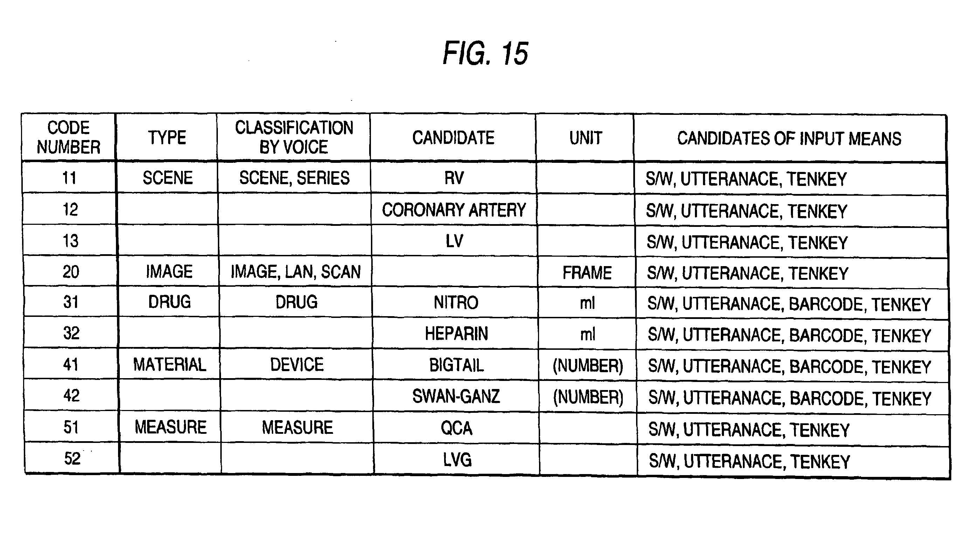

FIG. 14 shows a procedure for producing a bookmarked data file by the bookmarked data file-generating portion 19. The switch (S/W) 31, the microphone 22, the barcode reader 24, and the tenkey 25 are set as the input means for the bookmarked data files as shown in FIG. 15. One or any combination of these input means is used to enter information necessary to create bookmarked data files shown in FIGS. 13A-13E except for the date/time.

A type can be entered via the switch 31. Scan number, frame number, and amount can be entered via the tenkey 25. Device name and drug name can be entered via the barcode reader 24. All of these kinds of information can also be entered with the microphone 22 alone.

As shown in FIG. 14, if any signal is entered from these input means (S1), the signal is taken into the trigger signal judgment portion 26. Where the microphone 22 picks up speech (S2), the speech signal is converted into a text string by the speech recognition portion 23 (S3), and the text string data is supplied into the trigger signal judgment portion 26. The trigger signal judgment portion 26 makes a decision as to whether the input signal is an instruction for production of a bookmarked data file (S4). Where it is an instruction for producing a bookmarked data file, a trigger signal for producing a bookmarked data file is produced to the bookmarked data file-creating portion 29. This decision is made depending on whether the signal entered via the input means is coincident with the predetermined signal input rule.

As this signal input rule, "type" is first entered for every type. Subsequently to this entry of "type", "scene name" and so on are entered in the order shown in FIGS. 13A-13E. For example, it is previously determined that a type and the following contents are entered in the corresponding order such as "scene, RV", "image, scan 2, frame 31", "image, scan 3, frames 31 to 56", "drug, nitro, 10 ml", "device, Bigtail, French (4)", and "measure, scan 1, frame 12".

Accordingly, when a signal corresponding to any of the five types "scene", "image", "device", "drug", and "measure" is entered, its inputting can be judged to be a signal for instructing production of a bookmarked data file.

This signal input rule also contributes to improvement of the accuracy of speech recognition. Speech recognition tools are shown in FIG. 15. When any type signal of five types "scene", "image", "device", "drug", and "measure" is entered, candidates for the following input signal are displayed. For example, after "scene" is entered, "RV", "coronary artery", "LV", etc. are prepared as candidates for the scene name. Furthermore, after "drug" is entered, "nitro", "Heparin", etc. are prepared as candidates for the drug name. After "device" is entered, "Bigtail", "Swan-Ganz", etc. are prepared as candidates for the device name. The speech recognition error rates can be reduced by subjecting the results of speech recognition of these candidates to pattern matching.

Returning to FIG. 14, the bookmarked data file-creating portion 29 receiving the trigger signal makes a decision as to whether the input signal contains a special command (S5). This special command is prepared to facilitate entering "scan number" and "frame number" that are necessary for bookmarked data files for images and measurements. Usually, "scan number" and "frame number" are entered subsequently to inputting of "image" or "measure". A special command is entered instead of "scan number" and "frame number" subsequently to inputting of "image" or "measure".

"Now", "this image", "after n ^{·} seconds", "next heartbeat", etc. are prepared as examples of the special command. The bookmarked data file-creating portion 29 analyzes the input special command by referring to the table of special commands (S6), and supplies control data corresponding to the input special command to the inspection data-identifying information-gaining portion 28. The control data contains time data, for example, corresponding to the contents of the special command. To obtain inspection data-identifying information (scan number and/or frame number) corresponding to the time data, an access is made to the cardiovascular X-ray diagnostic system 2, inside pressure-measuring instrument 5, or the like, using the time data as a key (S7). Data about the obtained scan number and frame number is sent to the bookmarked data file-creating portion 29.

Then, the bookmarked information entered as described so far is converted into codes by the bookmarked data file-creating portion 29. In the bookmarked data file-creating portion 29, the type of the bookmarked data file is judged (S8). The type is converted into a code from the code table (S9). For example, if the type is a scene as shown in FIG. 15, a numeral in a range from ten to nineteen is assigned to the code. Similarly, a numeral in a range from twenty to twenty nine is assigned to an image. A numeral in a range from thirty to thirty nine is assigned to a drug. A numeral in a range from forty to forty nine is assigned to a device. A numeral in a range from fifty to fifty nine is assigned to a measurement (measure).

A number corresponding to a scene name, drug name, or device name is assigned to the least significant number of the code number. For example, "1" is assigned to RV, "3" is assigned to LV, "1" is assigned to nitro, and "2" is assigned to Swan-Ganz.

In a bookmarked data file, the type and the name of a scene or the like are expressed by two digits of numbers in this way.

Numeric data such as scan number, frame number, and amount are arranged subsequent to codes expressing the two digits of numbers that represent the type, the name of a scene, or the like. In this way, a bookmarked data file is created (S10). This created bookmarked data file is stored in the bookmarked data file database 13 via the bookmarked data file-writing portion 30 (S11).

The procedure for generating bookmarks from S1 to S11 as described so far is repeated up to the end of the inspection (S12). A plurality of bookmarked data files are produced and kept in storage.

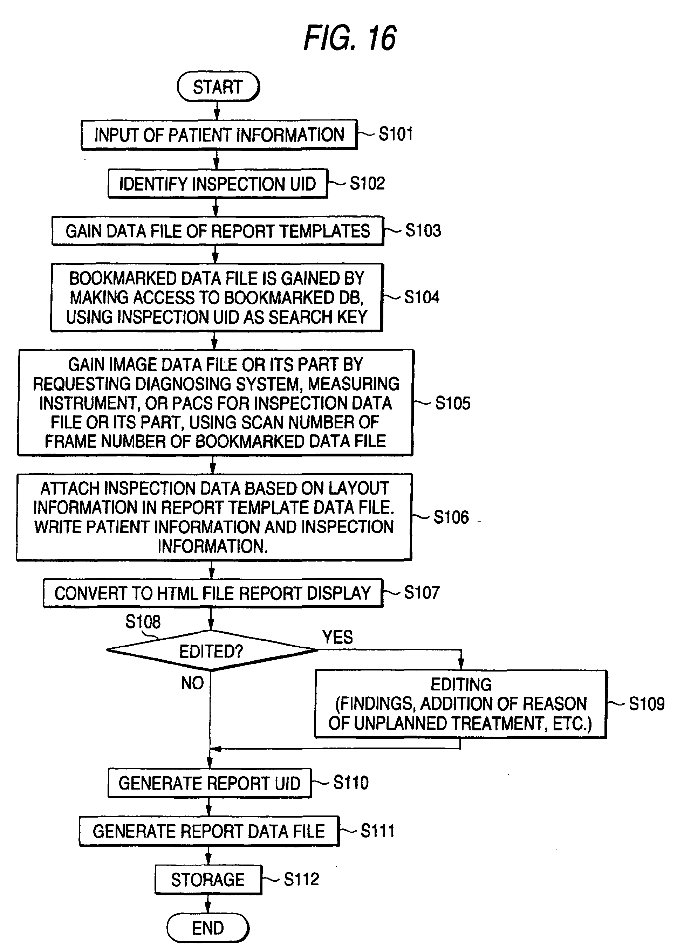

Then, the processing for creating an electronic report after the end of the inspection is described. The procedure for processing for creating the report is illustrated in FIG. 16. First, if patient information such as patient name and patient ID is entered via the keyboard 40 and so on (S101), the inspection list-gaining portion 32 routes the patient information to the inspection management portion 17. The inspection management portion 17 lists up information about one or more inspections of the patient conducted in the past. Also, the respective inspection UIDs and information discriminating whether a report has been created or not are sent back to the inspection list-gaining portion 32. In response to them, the information about one or more inspections of the patient conducted in the past is displayed on the display unit 15, together with the respective inspection UIDs and the information discriminating whether a report has been created or not. An inspection about which a report should be created at this time is selectively specified from within the list. Thus, an inspection UID about which the report should be created is specified (S102).

Then, the report template-gaining portion 33 sends a reply to a report template data file corresponding to the inspection to the inspection management portion 17, together with the identified inspection UID. The inspection management portion 17 refers to the inspection management table and sends back a report template data file corresponding to the inspection UID (S103). Inspection report templates for images, inspection report templates for measurements, report templates for devices, report templates for drugs, and report templates for recordings of execution are set in the report template data file. This report template data file is once placed into a working storage (not shown).

The bookmarked data file-gaining portion 34 gains access to the bookmarked data file DB 13, using the identified inspection UID as a search key, and gains all bookmarked data files associated with the inspection UID (S104). These bookmarked data files are once placed into a working storage (not shown).

Then, the inspection data file-gaining portion 35 selects files of image and scene types from the gained plural bookmarked data files, and extracts scan numbers and frame numbers from within the bookmarked data files. The gaining portion 35 requests the image database of the cardiovascular X-ray diagnostic system 2, the image database of the X-ray CT 3, or PACS 4 for plural inspection data files identified from the extracted scan numbers and frame numbers or parts thereof and gain them (S105). These inspection data files or data about some of them are once placed into a working storage (not shown).

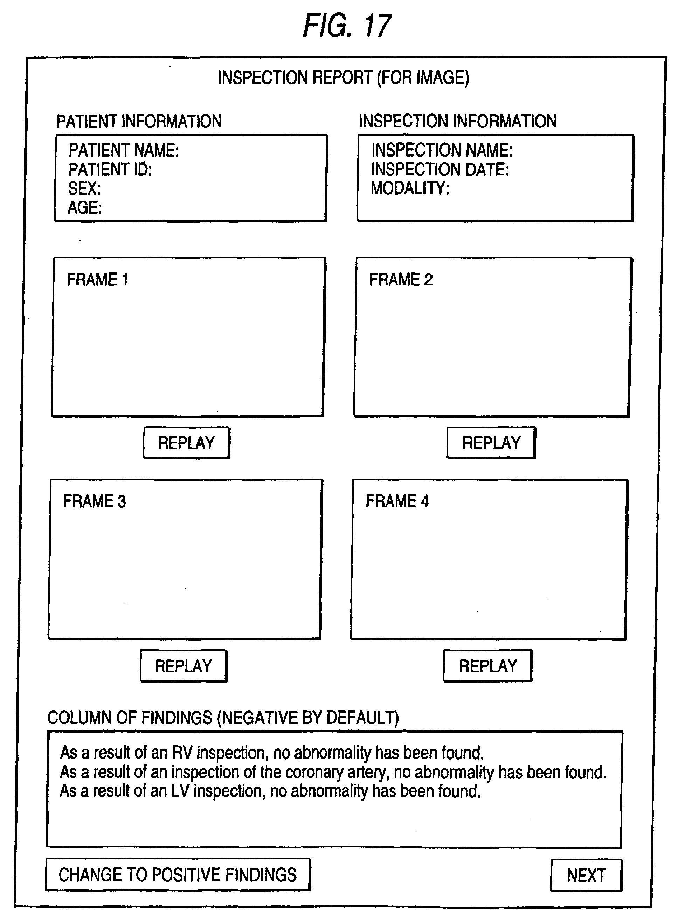

The gained inspection data is attached onto a report form according to the layout information in the inspection report template by the electronic report-creating portion 36. In addition, texts of patient information and inspection information are written into the report form (S106). FIG. 17 shows an example of inspection report for image. If there are plural bookmarked images, they are attached in turn into image attachment regions of inspection report templates for images according to the time. If there are plural bookmarked inside pressure waveform images, they are similarly attached in turn to the attachment regions of inspection report templates for measurements.

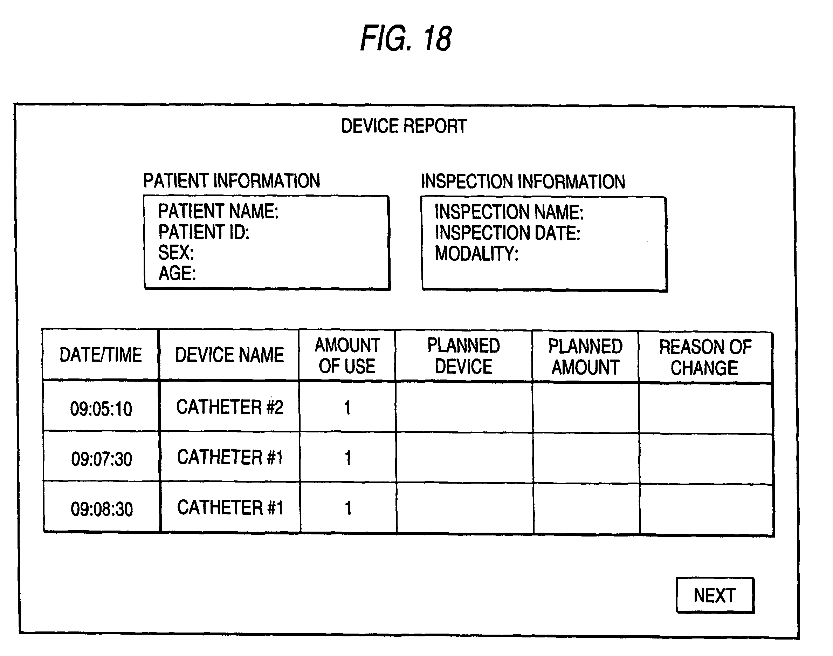

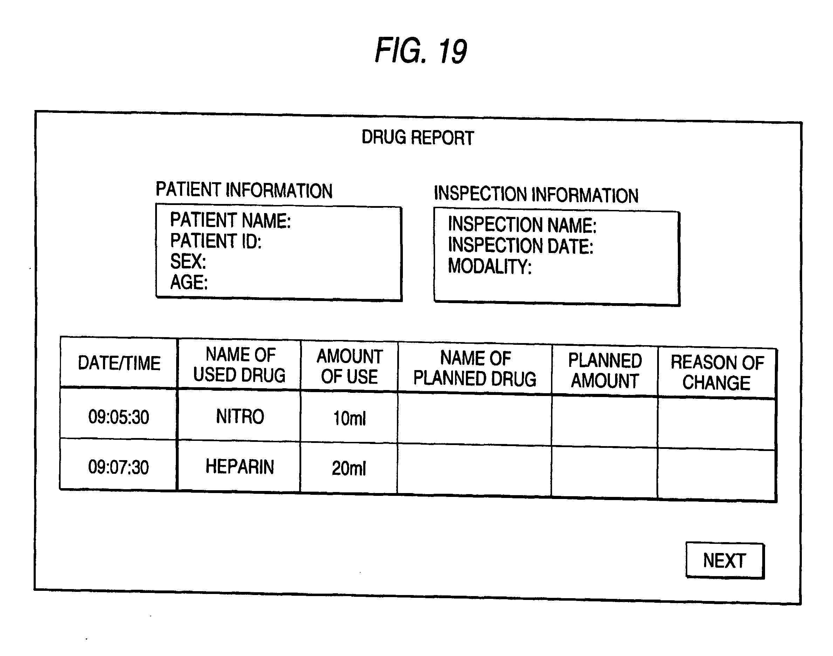

The electronic report-creating portion 36 extracts a bookmarked data file for devices and a bookmarked data file for drugs from all the bookmarked data files based on type data, arrays the extracted device names, drug names, and amounts according to the time, and writes texts of patient information and inspection information. Thus, as shown in FIGS. 18 and 19, a report for devices and a report for drugs are created.

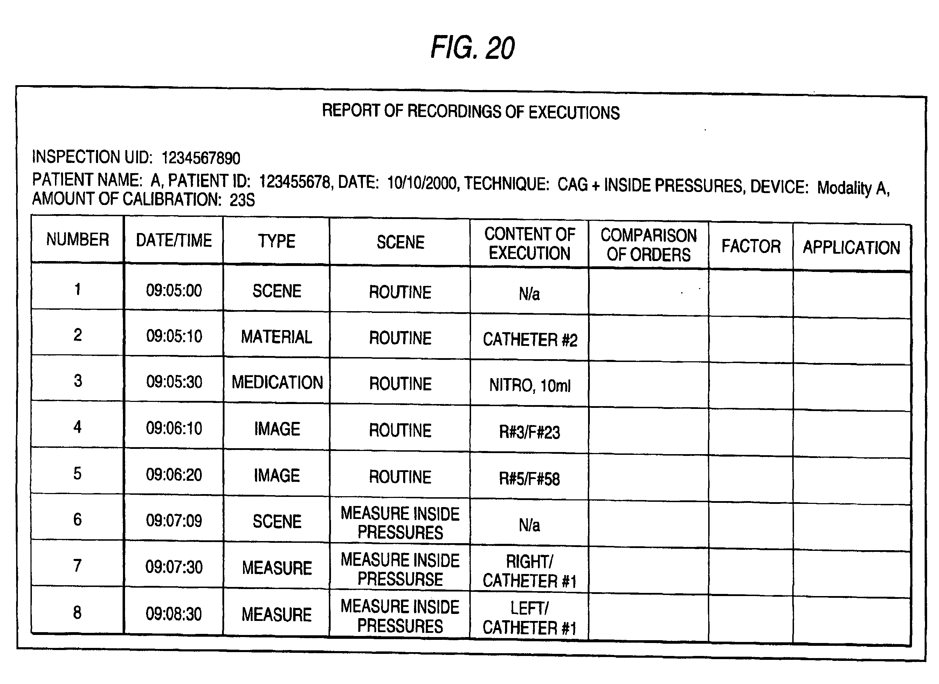

Furthermore, the electronic report-creating portion 36 arrays types, device names, drug names, and amounts according to the time for all the bookmarked data files. In addition, the creating portion writes texts such as of the patient information, inspection information, inspection names, and names of used inspection instruments, thus creating an execution-recording report as shown in FIG. 20.

These reports are original reports that are not yet finally edited. After making edits as described later, final reports are completed.

These original reports are displayed (S107). At this time, the original reports are converted into a file having high generality such as in HTML format. Furthermore, inspection data such as images and measurement waveforms is subjected to processing such as size reduction, compression, and file format conversion in the reference file-generating portion 38, and is converted into reference files. A report creator views the displayed reports and, if necessary, edits the reports under support of the editing functions of the report-editing portion 37 (S108 and S109).

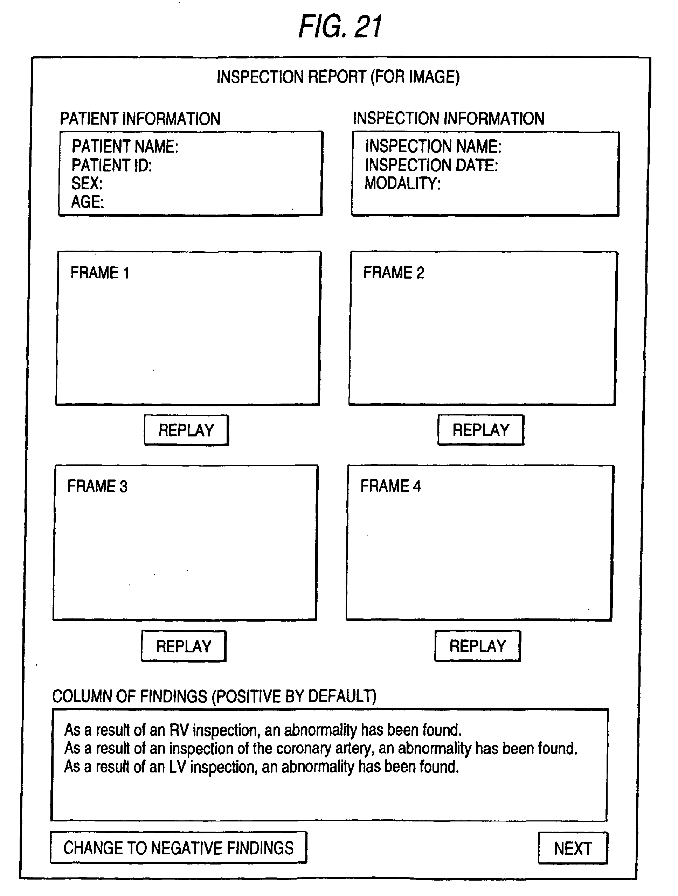

The main work of editing of the inspection reports is addition of findings. The report-editing portion 37 first displays default findings for negative findings as shown in FIG. 17. The default findings are created by connecting a clause such as "No abnormality has been found" to the scene name of the bookmarked data file for scene. The report creator appropriately modifies the default findings and adds a sentence. Positive findings are also prepared as the default findings. A switch is made from the default findings for negative findings to the default findings for positive findings shown in FIG. 21 in response to clicking of "change to positive findings" button. Such default findings contribute to improvement of the efficiency of the work for entering findings.

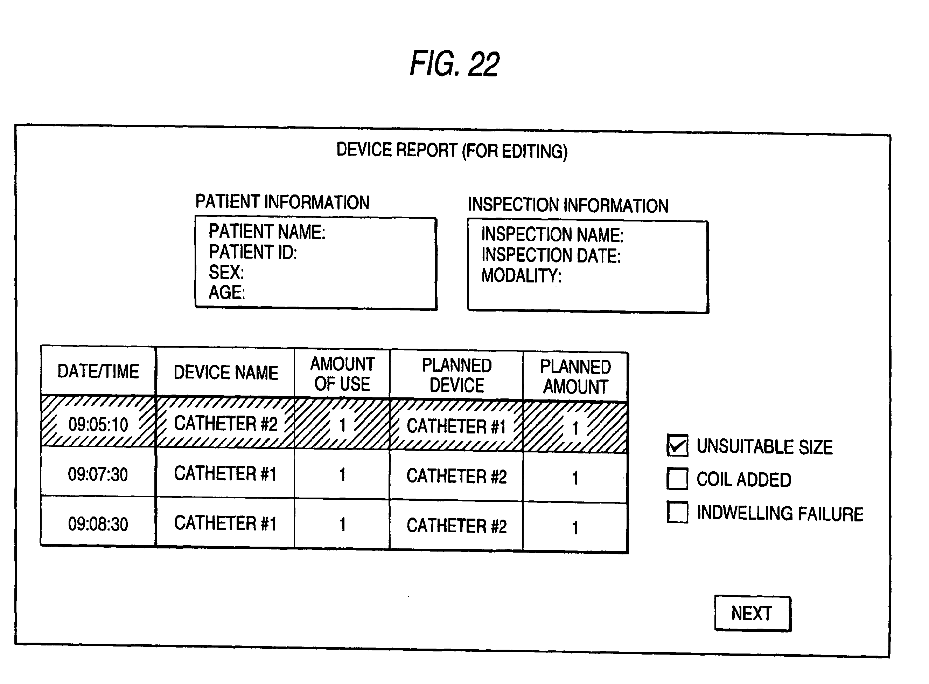

FIG. 22 shows an example of the editing screen of the device report. It is highly likely that a planned device is changed to a different kind of device by the judgment of the doctor in charge of the inspection or an unplanned device is used. Device names and the number taken from the device-specific, bookmarked data file are automatically entered into the original report. These are actually used devices and the planned number of used ones. The device report contains an entry column into which names of planned devices and the planned number are entered. The report creator enters the names of the planned devices and the number of them. Of course, these names of planned devices and the number of them can be extracted from the device set data file used when a device list was produced before inspection. Therefore, the editing portion 37 can automatically insert the planned devices and planned number in turn.

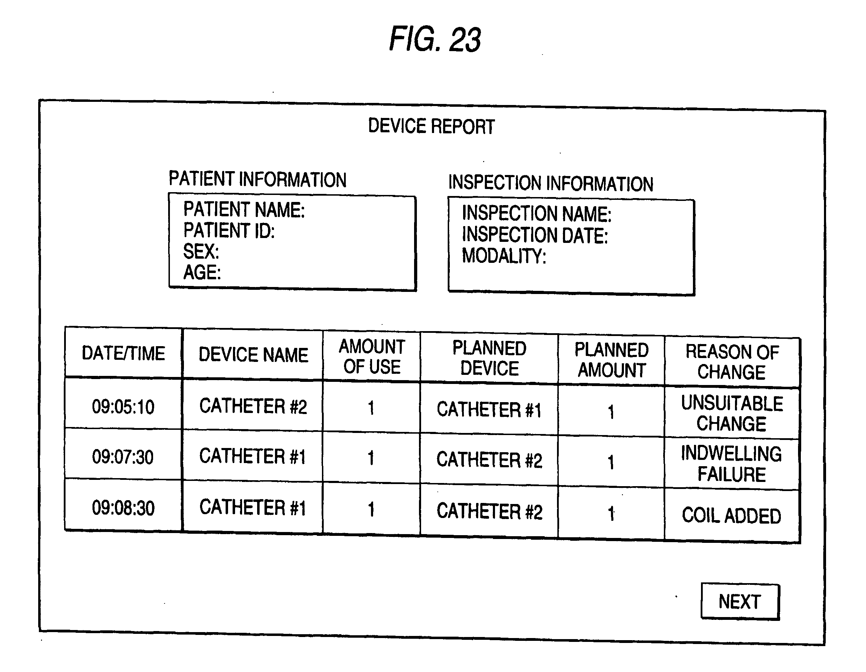

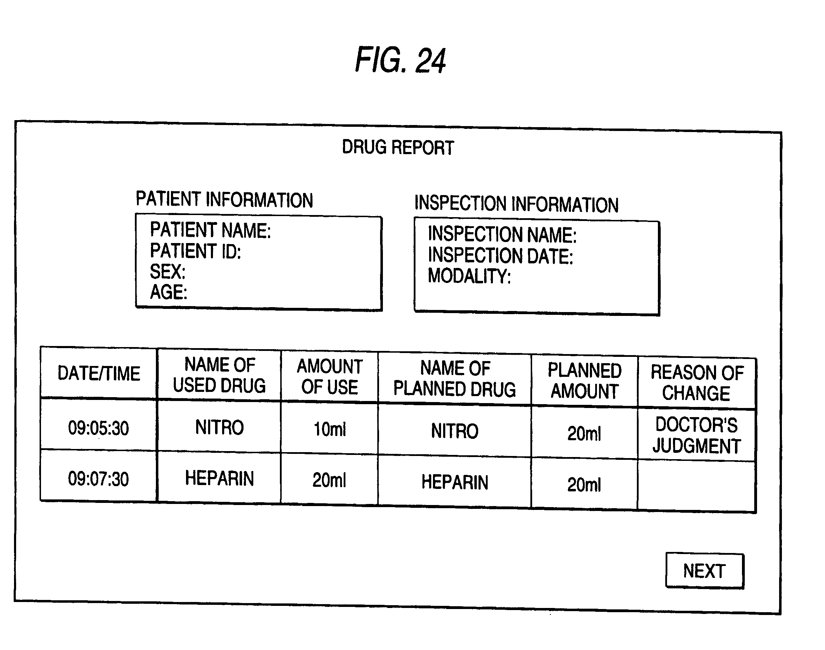

Candidates for plural reasons why devices or number different from the planned ones are used are displayed on the editing screen. If some row is selected, that row is displayed inverted. If any one of the candidates for the reasons of change (unsuitable size, addition of a coil, and indwelling failure or the like) is checked, the reason of change for the row is automatically is entered. An example of device report after an edit is shown in FIG. 23. A drug report is edited similarly. As shown in FIG. 24, planned drugs and planned amounts are automatically entered from the drug set data file. Also, with respect to the reason of change, it is automatically entered by a simple work consisting of making a choice out of candidates.

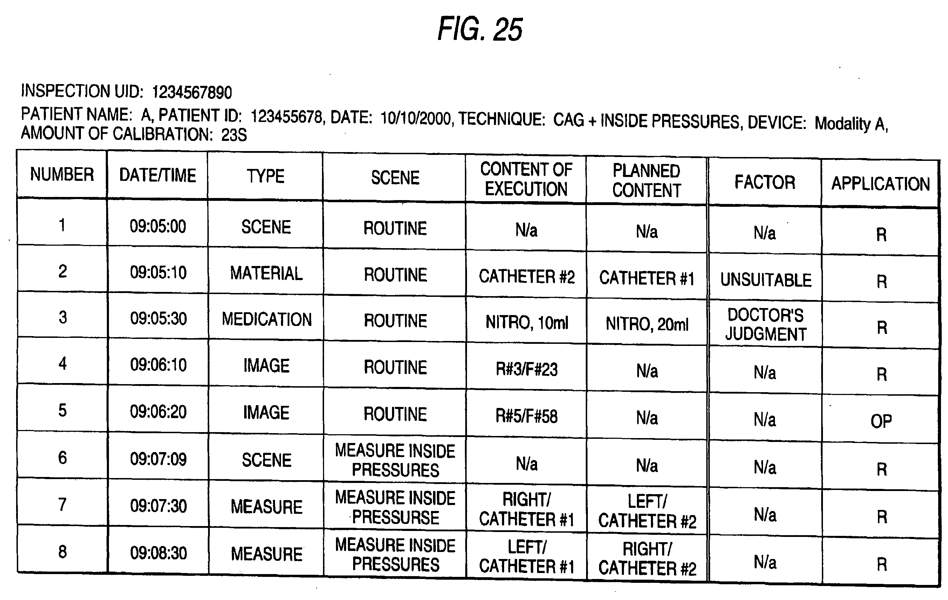

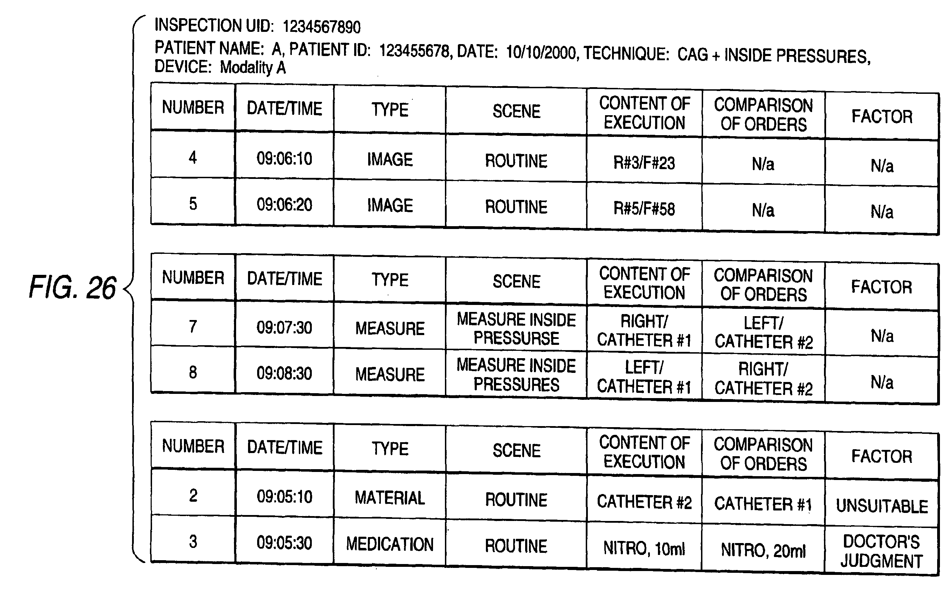

These device type change, amount change, and reason of change are reflected in the report of recording of execution as shown in FIG. 25. In the report of recording of execution, sorting can be done according to type as shown in FIG. 26.

After the end of the edit, a report UID for identifying the report data file is issued by the electronic report-creating portion 36 (S110). The report data file is converted into a file in HTML format. The reference file-generating portion 38 creates a reference file (S111). To check the report finally, the report data file in HTML format is decompressed and displayed by the browsing functions of the report viewer 39. When the bookmarked data file contains frame numbers, "Replay" button is displayed under the attachment region. Images or measurement waveforms are reproduced as moving pictures by clicking the button. Various buttons (not shown) for playback of some intervals, stoppage, and frame stepping are displayed together with the "Replay" button.

After the check, if an instruction for saving is given, the report data file is stored in the electronic report data file database 14 together with the reference file (S112).

As described so far, the present embodiment achieves great decrease in the labor for creating reports.

### Industrial Applicability

As described so far, the present invention is adapted to improve the creation of electronic reports.

## Claims

1. A system for assisting creation of an electronic report, the system being designed to assist creation of an electronic report data file about at least one inspection data file of interest or a part thereof, the inspection data file being contained in plural inspection data files produced by an inspection using a medical imaging diagnosing system or medical measuring instrument, said system for assisting creation comprising:
report template selection means for selecting a certain report template data file complying with an inspection purpose from plural report template data files containing layout information in said inspection data file;
bookmarked data file creation means for producing at least one bookmarked data file containing information identifying said inspection data file of interest or part thereof during said inspection;
inspection data file-accepting means for selectively taking in said inspection data file of interest or part thereof from said plural inspection data files based on said bookmarked data file;
electronic report data file-generating means for generating said electronic report data file using said inspection data file of interest or part thereof as a reference file, based on the layout information in said certain report template; and
means for outputting or storing said generated electronic report data file together with said reference file.

2. A system for assisting creation of an electronic report as set forth in claim 1, wherein said electronic report data file is an HTML file or a file in a similar format.

3. A system for assisting creation of an electronic report as set forth in claim 1, wherein said bookmarked data file-generating means has a microphone for converting uttered speech in accordance with given rules into a speech signal, a speech recognition portion for converting said speech signal into text string data, and a bookmarked data file-creating portion for creating said bookmarked data file based on said text string data.

4. A system for assisting creation of an electronic report as set forth in claim 3, wherein said text string data contains type of said inspection data file of interest and frame numbers of images.

5. A system for assisting creation of an electronic report as set forth in claim 4, wherein said bookmarked data file-generating means has means for gaining data about said frame numbers of images from said medical imaging disposing system or medical measuring instrument.

6. A system for assisting creation of an electronic report as set forth in claim 1, wherein said bookmarked data file contains information identifying said inspection data file of interest, an identification code for said inspection, date and time at which said bookmarked data file was produced, and type of said inspection data file of interest.

7. A system for assisting creation of an electronic report as set forth in claim 1, wherein said report template data file contains text files about said inspection and a patient undergoing said inspection and layout information about the text files, together with the layout information in said inspection data file.

8. A system for assisting creation of an electronic report as set forth in claim 1, wherein said electronic report data file-generating means has data-processing means for converting said inspection data file of interest into said reference file based on said layout information.

9. A system for assisting creation of an electronic report as set forth in claim 1, wherein said electronic report data file-generating means has editing means for editing said electronic report data file.

10. A system for assisting creation of an electronic report as set forth in claim 1, wherein said bookmarked data file-generating means produces type-specific bookmarked data files about drugs or devices used during said inspection besides type-specific bookmarked data files about said inspection data files.

11. A system for assisting creation of an electronic report as set forth in claim 10, wherein said type-specific bookmarked data files about drugs or devices contain data about names of said drugs or devices, date and time of use, and their amounts of use.

12. A system for assisting creation of an electronic report as set forth in claim 11, wherein said electronic report data file-generating means produces report data files of use history of said drugs or devices based on said type-specific bookmarked data files about drugs or devices.

13. A system for assisting creation of an electronic report as set forth in claim 11, further including a barcode reader for reading barcodes printed on said drugs or devices.

14. A method of assisting creation of an electronic report data file about at least one inspection data file of interest or a part thereof by the use of a computer, the inspection data file being contained in plural inspection data files produced by an inspection using a medical imaging diagnosing system or medical measuring instrument, said method comprising the steps of:
selecting a certain report template data file complying with an inspection purpose from plural report template data files containing layout information in said inspection data file;
producing at least one bookmarked data file containing information identifying said inspection data file of interest or part thereof during said inspection;
selectively taking in said inspection data file of interest or part thereof from said plural inspection data files based on said bookmarked data file;
producing said electronic report data file using said inspection data file of interest or part thereof as a reference file based on layout information in said certain report template data file; and
outputting or storing said produced electronic report data file together with said reference file.

15. A method of assisting creation of an electronic report as set forth in claim 14, wherein said step of producing said bookmarked data file includes the substeps of converting a speech signal entered via a microphone into text string data and creating said bookmarked data file based on said text string data.

16. A method of assisting creation of an electronic report as set forth in claim 14, wherein said step of producing said bookmarked data file includes the substep of gaining information identifying said inspection data file of interest or part thereof from said medical imaging diagnosing system or medical measuring instrument.

17. A program for assisting creation of an electronic report by operating a computer to assist creation of an electronic report data file about at least one inspection data file of interest or a part thereof, the inspection data file being contained in plural inspection data files produced by an inspection using a medical imaging diagnosing system or medical measuring instrument such that the computer acts as:
means for selecting a certain report template data file complying with an inspection purpose from plural report template data files containing layout information in said inspection data file;
means for producing at least one bookmarked data file containing information identifying said inspection data file of interest or part thereof during said inspection;
means for selectively taking in said inspection data file of interest or part thereof from said plural inspection data files based on said bookmarked data file;
means for producing said electronic report data file using said inspection data file of interest or part thereof as a reference file based on layout information in said certain report template data file; and
outputting or storing said produced electronic report data file together with said reference file.

18. A program as set forth in claim 17, wherein said means for producing the bookmarked data file includes means for converting a speech signal entered via a microphone into text string data and means for creating said bookmarked data file based on said text string data.

19. A program as set forth in claim 17, wherein said means for producing the bookmarked data file includes means for gaining information identifying said inspection data file of interest or part thereof from said medical imaging diagnosing system or medical measuring instrument.
